# EUROPEAN PATENT APPLICATION

(11) **EP 4 768 589 A1**
(43) Date of publication of application: **01.07.2026**
(21) Application number: 24929879.5
(22) Date of filing: 05.11.2024
(51) Int. Cl.: C12N 15/67, C12N 15/77, C12N 15/70, C12N 15/75, C12P 13/08, C12P 13/06

(54) **NOVEL PROMOTER AND USES THEREOF**

(30) Priority: 13.03.2024 KR 20240035063
(71) Applicant: CJ Cheiljedang Corporation, Seoul 04560 (KR)
(72) Inventor: YOON, Jeong-Hye, Seoul 04560 (KR); PARK, Goun, Seoul 04560 (KR); BAE, Jee Yeon, Seoul 04560 (KR); KIM, Hyung Joon, Seoul 04560 (KR)
(74) Representative: Meissner Bolte Partnerschaft mbB
(86) International application number: PCT/KR2024/017294
(87) International publication number: WO 2025/192818

(57) **Abstract**

The present application relates to a novel promoter and a method for producing a target product using same. A polynucleotide according to one embodiment has promoter activity, can be introduced into a microorganism to enhance the expression and activity of a gene operably linked thereto, and can be effectively used for efficiently producing a target product that is affected by the polynucleotide and the gene.

## Description

### [TECHNICAL FIELD]

### Cross-reference to prior application(s)

The present application claims the benefit of the priority based on Korean Patent Application No. 10-2024-0035063 filed on March 13, 2024, and the entire contents disclosed in the document of the corresponding Korean patent application are incorporated as a part of the present description.

The present application relates to a novel promoter, and a method for producing a target product using the same.

### [BACKGROUND ART]

In order to produce target substances such as amino acids or useful substances or the like that can be used for various uses such as feed, medicine, food, and the like using microorganisms at high titer, efforts such as genetic manipulation and/or external gene introduction and the like in biosynthetic pathways have been continuously made. As one of such methods, there is a method for inducing overexpression of a target gene in a microorganism, and for this, a highly efficient gene expression system is required. A promoter is one of factors that are greatly involved in the expression level and expression regulation of a gene, so it can be said that development of a useful promoter is essential for developing an expression system.

The tac promoter derived from E. coli is widely known as a strong promoter, and in case of coryneform microorganisms, strong promoters have been developed by modifying promoters of their own genes (Gene, 102, 93-98, 1991; Microbiology, 142, 1297-1309, 1996). On the other hand, the general structure of the promoter sequence for gene expression in the coryneform microorganisms is not known, unlike other industrial microorganisms such as *E. coli* or *Bacillus subtilis.* Therefore, promoters have been developed by measuring the antibiotic resistance of strain obtained by removing the promoter portion of antibiotic resistance genes such as chloramphenicol, introducing chromosomal DNA isolated from coryneform microorganisms by cutting it with an appropriate restriction enzyme, and then transforming the coryneform microorganisms with it. In addition, exploration of various promoters has been conducted to overexpress foreign genes in microorganisms of the genus *Bacillus* until now, and production of food, pharmaceutical and other industrial enzymes using this has been conducted. Many vector systems using expression promoters of alpha-amylase, protease and lipase genes in various microorganisms of the genus *Bacillus* have been continuously made up to now (Schumann 2007. Adv. Appl. Microbiol. 153:813-821).

However, since a system exhibiting high expression efficiency various microorganisms, namely, even in microorganisms of the genus *Escherichia,* microorganisms of the genus *Corynebacterium,* or microorganisms in the genus *Bacillus,* or the like is still needed, a need for development of a universal promoter is still emerging.

### [PRIOR ARTS]

### [PATENT DOCUMENTS]

(Patent document 1) U.S. Granted Patent (US 11,041,181 B2)

### [DISCLOSURE]

### [TECHNICAL PROBLEM]

One object of the present application is to provide a novel polynucleotide.

Another object of the present application is to provide an expression cassette comprising the polynucleotide and a target gene.

Other object of the present application is to provide a recombinant vector (expression vector) comprising the polynucleotide; or the expression cassette.

Other object of the present application is to provide a microorganism, comprising at least one selected from the group consisting of the polynucleotide, an expression cassette comprising the polynucleotide and a target gene, and a vector comprising the expression cassette.

Other object of the present application is to provide a method for producing a target product comprising culturing the microorganism in a medium. The method may further comprise recovering the target product from the medium or the microorganism resulting from the culturing, after the culturing.

Other object of the present application is to provide a composition for producing a target product comprising the microorganism, a medium in which the microorganism is cultured, or a combination thereof.

Other object of the present application is to provide a use of the polynucleotide as a promoter.

Other object of the present application is to provide a use of the microorganism, a medium in which the microorganism is cultured, or a combination thereof for producing a target product.

### [TECHNICAL SOLUTION]

Each description and embodiment disclosed in the present description may be also applied to each other description and embodiment. In other words, all combinations of various elements disclosed in the present description fall within the scope of the present application. In addition, the scope of the present application cannot be considered limited by the specific description described below.

Hereinafter, it will be described in more detail.

One aspect of the present application provides a novel polynucleotide.

Another aspect provides the use of the polynucleotide as a promoter.

**In** the present description, "polynucleotide" comprises nucleotide monomers of 2 or more, 5 or more, 10 or more, 13 or more, 20 or more, or 30 or more, and the nucleotide monomers may be covalently bonded to form a chain.

**In** one embodiment of the present application, the polynucleotide may be a polynucleotide comprising a nucleic acid sequence in which the 19th nucleotide, the 22nd nucleotide, or both, from the 5' end of the nucleic acid sequence of SEQ **ID** NO: 1 are substituted with other nucleotides.

**In** one embodiment, the polynucleotide may comprise a nucleic acid sequence in which the 19th nucleotide from the 5' end of the nucleic acid sequence of SEQ **ID** NO: 1 is substituted with a different nucleotide (e.g., adenine (A), thymine (T), or cytosine (C)).

**In** one embodiment, the polynucleotide may comprise a nucleic acid sequence in which the 21st nucleotide from the 5' end of the nucleic acid sequence of SEQ **ID** NO: 1 is substituted with a different nucleotide (e.g., thymine (T), guanine (G), or cytosine (C)).

**In** one embodiment, the polynucleotide may comprise a nucleic acid sequence in which the 19th nucleotide from the 5' end of the nucleic acid sequence of SEQ **ID** NO: 1 is substituted with a different nucleotide (e.g., adenine (A), thymine (T), or cytosine (C)), and
the 21st nucleotide from the 5' end of the nucleic acid sequence of SEQ **ID** NO: 1 is substituted with a different nucleotide (e.g., thymine (T), guanine (G), or cytosine (C)).

**In** one embodiment, the polynucleotide may comprise a nucleic acid sequence selected from the group consisting of SEQ **ID** NO: 39 and SEQ **ID** NO: 40.

**In** one embodiment, the polynucleotide may comprise a nucleic acid sequence in which the 21st nucleotide from the 5' end of the nucleic acid sequence of SEQ **ID** NO: 1 is substituted with cytosine.

The polynucleotide may comprise the nucleic acid sequence of SEQ **ID** NO: 39.

**In** one embodiment, the polynucleotide may comprise a nucleic acid sequence in which the 19th nucleotide from the 5' end of the nucleic acid sequence of SEQ **ID** NO: 1 is substituted with adenine.

The polynucleotide may comprise the nucleic acid sequence of SEQ ID NO: 40.

In one embodiment, the polynucleotide may comprise a nucleic acid sequence in which the 19th nucleotide from the 5' end of the nucleic acid sequence of SEQ ID NO: 1 is substituted with adenine, and the 21st nucleotide from the 5' end of the nucleic acid sequence of SEQ ID NO: 1 is substituted with cytosine.

The polynucleotide may be a polynucleotide in which 3 to 6 nucleotides are added to the 3' end of the nucleic acid sequence of SEQ ID NO: 1.

In one embodiment, the polynucleotide may be a polynucleotide in which three nucleotides "CAT" (5'-CAT-3') are added to the 3' end of the nucleic acid sequence of SEQ ID NO: 1.

In one embodiment, the polynucleotide may be a polynucleotide in which six nucleotides "ACTAGT" (5'-ACTAGT-3') are added to the 3' end of the nucleic acid sequence of SEQ ID NO: 1.

In one embodiment, the polynucleotide may comprise the nucleic acid sequence of SEQ ID NO: 41 or SEQ ID NO: 42.

The polynucleotide may have promoter activity and/or may be used as a universal promoter.

In one embodiment, the polynucleotide may have promoter activity for expression in microorganisms of the genus *Corynebacterium,* microorganisms of the genus *Escherichia,* and/or microorganisms of the genus *Bacillus.*

In the present specification, the nucleic acid sequence of the Po2 promoter (U.S. Patent No. US 10,273,491 B2) is shown in SEQ ID NO: 1.

The polynucleotide according to one specific embodiment may be used as a synthetic promoter having strong expression-inducing activity, and for example, may express a target gene in microorganisms of the genus *Corynebacterium,* microorganisms of the genus *Escherichia,* and/or microorganisms of the genus *Bacillus* with significantly higher efficiency than the conventionally used Po2 promoter.

The polynucleotide according to one specific embodiment may be natural or non-natural, and for example, it may be non-natural, chemically or recombinantly synthesized.

In the present description, "promoter" may mean a DNA region which comprises a binding site to polymerase and allows to initiate transcription of a target nucleotide sequence in the downstream. The promoter may be located at the 5' region of the transcription initiation site. The polynucleotide may be linked upstream (toward the 5' end) and/or downstream (toward the 3' end) of a target gene, and the polynucleotide may be linked to the target gene operably and/or in a manner that allows for regulation (enhancement or attenuation) of expression. For example, the polynucleotide may be linked to the 5' end of a target gene to enhance (increase) expression of the target gene, or may be linked to the 3' end of the target gene to attenuate (decrease) expression of the target gene.

In one embodiment, for the purpose of attenuating expression of a target gene, a promoter may be introduced in a reverse orientation downstream of the stop codon of the target gene, preferably between the stop codon and an upstream region of a transcription terminator. The target gene is transcribed in the reverse direction such that RNA polymerase complexes collide during transcription, thereby attenuating expression of the target gene.

The polymerase is also called RNA polymerase, or DNA dependent RNA polymerase, and may refer to an enzyme which synthesizes primary transcript RNA. The polymerase may be RNA polymerase of prokaryotic cells, or RNA polymerase of eukaryotic cells (for example, RNA polymerase I, RNA polymerase II, RNA polymerase III, RNA polymerase IV or RNA polymerase V, etc.).

In the present description, "target gene" may refer to a gene to be expressed. In one embodiment, the target gene may refer to a gene encoding a target protein.

The target protein may be a protein (for example, enzyme) involved in production of a target product.

In the present specification, "target product" may refer to a bioactive substance, for example, one or more selected from the group consisting of amino acids, amino acid derivatives, nucleic acids, nucleic acid derivatives, vitamins, vitamin derivatives, sugars, sugar derivatives, fatty acids, fatty acid derivatives, proteins, and other metabolites.

In the present description, the term "target product" may mean a biologically active substance to be finally produced or regulate (increase or reduce) production using the polynucleotide provided in the present description, an expression cassette, an expression vector, and/or a recombinant cell, comprising the same, and for example, it may mean the target protein encoded by the target gene itself, and/or all biologically active substances produced as the target protein involves therein. The biologically active substances mean all substances which is produced or derived from an organism (for example, cell) or has a certain function in a living body or cell, and for example, it may be at least one selected from the group consisting of amino acids, amino acid derivatives, nucleic acids (adenine, thymine, guanine, cytosine, uracil, etc.), nucleic acid derivatives, vitamins (vitamin A (retinol), B (B1 (thiamine), B2 (riboflavin), B3 (niacin), B5 (pantothenic acid), B6 (pyridoxine), B7, B9 (folic acid), B12 (cobalamin), etc.), C (ascorbic acid), D (calciferol), E (tocopherol), K (phylloquinone), etc.), vitamin derivatives, proteins (proteins other than the target protein, for example, hormones, growth factors, cytokines, immunoglobulins (antibodies), antigen proteins, receptors, ligands, functional fragments (fragments having targeted functions) thereof, fusion proteins in which at least 2 kinds are fused, etc.), sugars (for example, monosaccharides (glyceraldehyde, dihydroxy acetone, erythrose, threose, erythrulose, arabinose, lyxose, ribose, xylose, ribulose, xylulose, deoxyribose, allose, altrose, galactose, glucose, gulose, idose, mannose, talose, fructose, psicose, sorbose, tagatose, fucose, fuculose, rhamnose, mannoheptulose, sedoheptulose, etc.), disaccharides (cellobiose, isomaltose, isomaltulose, lactose, lactulose, maltose, sucrose, trehalose, turanose, etc.), polysaccharides, etc.), sugar derivatives (sugar alcohol, galactosamine, glucosamine, sialic acid, N-acetylglucosamine, sulfoquinovose, ascorbic acid, mannitol, glucuronic acid, etc.), fatty acids (myristoleic acid, palmitoleic acid, sapienic acid, oleic acid, elaidic acid, vaccenic acid, linoleic acid, linolelaidic acid, arachidonic acid, eicosapentaenoic acid (EPA), erucic acid, docosahexaenoic acid (DHA), etc.), fatty acid derivatives, organic acids (lactic acid, citric acid, oxalic acid, uric acid, butyric acid, stearic acid, propionic acid, etc.), metabolites thereof (polyhydroxyalkanoate (PHA), etc.), precursors thereof, derivatives maintaining biological activity thereof, and the like, but is not limited thereto.

In one embodiment, when the target protein is involved in production of the target product, (1) the target protein may be a protein involved in at least one process or step of intracellular pathway (for example, biosynthesis, metabolism, bioconversion, etc.), intracellular transport, and/or exocytosis pathway of the target product, for example, at least one selected from the group consisting of synthetase, lyase, kinase, carboxylase (e.g., pyruvate carboxylase, etc.), reductase, oxidase, decarboxylase, dehydrogenase, dehydratase, transferase (e.g., transferase), epimerase, etc.), intermediate products, carrier proteins, membrane proteins (channels, etc.) and the like, but is not limited thereto, and
(2) the target gene may be a gene encoding the target protein described in (1) above.

The amino acid in the target product, may be a proteinogenic amino acid, or non-proteinogenic amino acid.

The proteinogenic amino acid may be at least one selected from the group consisting of arginine, histidine, lysine, aspartic acid, glutamic acid, serine, threonine, asparagine, glutamine, cysteine, glycine, proline, selenocysteine, pyrrolysine, alanine, valine, isoleucine, leucine, methionine, phenylalanine, tyrosine and tryptophan.

The L-amino acid among the target products is not particularly limited. That is, any L-amino acid that can be produced from a microorganism may be included without limitation, and intermediates of the L-amino acid may also be included. The L-amino acid may be, for example, at least one selected from the group consisting of L-arginine, L-histidine, L-lysine, L-aspartic acid, L-glutamic acid, L-serine, L-threonine, L-asparagine, L-glutamine, L-tyrosine, L-alanine, L-isoleucine, L-leucine, L-valine, L-phenylalanine, L-methionine, L-tryptophan, L-glycine, L-proline and L-cysteine, and specifically, it may be L-lysine, L-threonine, L-isoleucine, L-leucine, L-valine, L-arginine, or L-glutamic acid, but is not limited thereto. The intermediate of the L-amino acid may be for example, O-acetyl homoserine, but is not limited thereto.

The proteinogenic amino acid may be at least one selected from the group consisting of beta-alanine (β-alanine), gamma-aminobutyric acid (γ-Aminobutyric acid, GABA), 5-aminolevulinic acid (δ-Aminolevulinic acid), p-aminobenzoic acid (4-Aminobenzoic acid), alpha-aminobutyric acid (α-aminoisobutyric acid), dehydroalanine, cystathionine, lanthionine, djenkolic acid, diaminopimelic acid, Norvaline, norleucine, alloisoleucine, t-leucine (tert-Leucine), alpha-amino-n-heptanoic acid (α-amino-n-heptanoic acid), pipecolic acid, alpha, beta-diaminopropionic acid (α,β-diaminopropionic acid), alpha-gamma-diaminobutyric acid (α,γ-diaminobutyric acid), ornithine, allothreonine, homocysteine, homoserine (or isothreonine), O-acetyl homoserine, and the like.

The amino acid may be a D-amino acid or L-amino acid.

In one specific embodiment, the target product may be at least one selected from the group consisting of threonine, O-acetyl homoserine and valine.

The polynucleotide of the present application according to one specific embodiment may regulate (for example, increase or decrease) expression of a target gene operably linked thereto, production and/or activity of a target protein encoded by the target gene, and/or production and/or activity of a biologically active substance involved in production of the target protein, compared to a conventional promoter or cell-intrinsic promoter, in a cell.

The nucleic acid sequence of the polynucleotide of the present application may be modified by conventionally known mutagenesis, for example, direct evolution and site-directed mutagenesis and the like. In other words, the polynucleotide may comprise or consist of a nucleic acid sequence having homology or identity of 60% or more, 65% or more, 70% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 90.5% or more, 91% or more, 91.5% or more, 92% or more, 92.5% or more, 93% or more, 93.5% or more, 94% or more, 94.5% or more, 95% or more, 95.5% or more, 96% or more, 96.5% or more, 97% or more, 97.5% or more, 98% or more, 98.5% or more, 99% or more, 99.5% or more or 99.9% or more to the nucleic acid sequence of SEQ ID NO: 1. Any polynucleotide having substantially the same or corresponding biological activity as the nucleic acid sequence of SEQ ID NO: 1 is included within the scope of the present application, even if the polynucleotide comprises a nucleotide sequence in which part of the sequence of SEQ ID NO: 1 is deleted, modified, substituted, or added.

In the present description, the term "homology" means the degree of matching with a given nucleic acid sequence or amino acid sequence, and may be expressed as a percentage (%). For example, the homology may be determined by directly aligning sequence information between two polynucleotide molecules or two polypeptide molecules, for example, parameters such as score, identity and similarity and the like, using an easily available computer program aligning sequence information. The computer program may be BLAST (NCBI), CLC Main Workbench (CLC bio), MegAlignTM (DNASTAR Inc) and the like.

In one specific embodiment, the polynucleotide comprising a specific nucleic acid sequence provided in the present description may be interpreted to comprise not only the specific nucleic acid sequence or a nucleic acid sequence substantially equivalent thereto, but also a nucleic acid sequence complementary to the specific nucleic acid sequence. Specifically, the complementary polynucleotide may be hybridized at a Tm value appropriately adjustable by those skilled in the art according to the purpose, for example, at a Tm value of 55°C, 60°C, 63°C or 65°C, and be analyzed under conditions described later: such conditions are specifically described in known documents. For example, a condition in which genes with a high complementarity of 60% or more, 70% or more, 80% or more, 85% or more, 90% or more, 91% or more, 92% or more, 93% or more, 94% or more, 95% or more, 96% or more, 97% or more, 98% or more, 98% or more, 99.5% or more, or 99.9% or more are hybridized, and genes having a lower complementarity are not hybridized, or a condition of washing once, specifically, twice or three times, at a salt concentration and temperature corresponding to 60°C, 1x SSC(saline-sodium citrate buffer), and 0.1%(w/v) SDS (Sodium Dodecyl Sulfate); 60°C, 0.1x SSC, and 0.1% SDS; or 68°C, 0.1x SSC, and 0.1% SDS, which is a washing condition of common southern hybridization, and the like may be listed, but is not limited thereto. Hybridization may require two nucleotides to have complementary sequences, or base mismatches may be allowed depending on the stringency of the hybridization. The term, "complementary" may be used to describe relationship between nucleotide bases capable of hybridizing to each other. For example, in case of DNA, adenosine is complementary to thymine, and cytosine is complementary to guanine. The suitable stringency to hybridize a polynucleotide depends on the length and degree of complementarity of the polynucleotide, and this is well known in the technical field related thereto (See Sambrook et al., supra,9.50-9.51, 11.7-11.8).

According to one specific embodiment, the polynucleotide may comprise the nucleic acid sequence of SEQ ID NO: 39 or SEQ ID NO: 40 and may also refer to a polynucleotide consisting of, or essentially comprising, the nucleic acid sequence of SEQ ID NO: 39 or SEQ ID NO: 40, and/or a polynucleotide comprising a nucleic acid sequence having homology or identity of 60% or more, 65% or more, 70% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 90.5% or more, 91% or more, 91.5% or more, 92% or more, 92.5% or more, 93% or more, 93.5% or more, 94% or more, 94.5% or more, 95% or more, 95.5% or more, 96% or more, 96.5% or more, 97% or more, 97.5% or more, 98% or more, 98.5% or more, 99% or more, 99.5% or more, or 99.9% or more to the nucleic acid sequence of SEQ **ID** NO: 39 or SEQ **ID** NO: 40, while maintaining the original function and/or an desired function of the polynucleotide.

**In** the present specification, the expression "a polynucleotide consisting of the nucleic acid sequence of SEQ ID NO: ~" does not exclude addition, deletion, and/or mutation of nucleotides that may occur during the process of linking the polynucleotide to a target gene when used as a promoter according to one specific embodiment, for example during restriction enzyme-mediated cloning.

**In** the present description, that a polynucleotide or polypeptide "comprises a specific nucleic acid sequence or amino acid sequence" may refer to that the polynucleotide or polypeptide consists of the specific nucleic acid sequence or amino acid sequence or essentially comprises thereof, and it may be interpreted to include "a substantially equivalent sequence" in which a mutation (deletion, substitution, modification, and/or addition) is applied to the specific nucleic acid sequence or amino acid sequence within a range of maintaining an original function and/or desired function of the polynucleotide or polypeptide (or not exclude the mutation). **In** one embodiment, the nucleic acid sequence or amino acid sequence provided in the present description may include those modified by common mutagenesis, for example, direct evolution and/or site-directed mutagenesis and the like in a range of maintaining an original function or desired function thereof. **In** one specific embodiment, that a polynucleotide or polypeptide "comprises a specific nucleic acid sequence or amino acid sequence" may refer to that the polynucleotide or polypeptide (i) consists of the specific nucleic acid sequence or amino acid sequence, or essentially comprises thereof, or (ii) consists of an amino acid sequence having homology or identity of 60% or more, 65% or more, 70% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 90.5% or more, 91% or more, 91.5% or more, 92% or more, 92.5% or more, 93% or more, 93.5% or more, 94% or more, 94.5% or more, 95% or more, 95.5% or more, 96% or more, 96.5% or more, 97% or more, 97.5% or more, 98% or more, 98.5% or more, 99% or more, 99.5% or more or 99.9% or more to the specific nucleic acid sequence or amino acid sequence or essentially comprises thereof and maintains the original function and/or desired function.

According to one specific embodiment, the original and/or desired function of the polynucleotide may be a function as a promoter. The fact that the polynucleotide has a function as a promoter and comprises the nucleic acid sequence of SEQ ID NO: 39 or SEQ ID NO: 40 may mean that it does not exclude cases where mutations, such as addition, deletion, and/or substitution of nucleotides, which may occur during the process of linking to a target gene (e.g., through the use of restriction enzymes), are induced (introduced) into the nucleic acid sequence of SEQ ID NO: 39 or SEQ ID NO: 40 when the polynucleotide is operably linked to the target gene as a promoter. Furthermore, the polynucleotide comprising the nucleic acid sequence of SEQ ID NO: 39 or SEQ ID NO: 40 and having promoter activity may include, without limitation, any polynucleotide that hybridizes under stringent conditions with all or part of the nucleic acid sequence of SEQ ID NO: 39 or SEQ ID NO: 40, or a sequence complementary thereto, and possesses promoter activity.

The polynucleotide may be operably linked to a gene encoding a target protein.

As described above, the target protein may be involved in the production of a target product, and examples thereof are as follows.

In one embodiment, the protein involved in production of arginine among the amino acids may be at least one selected from proteins involved in arginine biosynthesis (arginine biosynthesis proteins). For example, the protein involved in arginine biosynthesis may be N-acetylglutamate synthetase (argA), N-acetylglutamate kinase (argB), N-acetylglutamyl phosphate reductase (argC), acetyl ornithine aminotransferase (argD), acetyl ornithine deacetylase (argE), ornithine carbamoyltransferase (argF, argI), argininosuccinate synthetase (argG), argininosuccinate lyase (argH), ornithine acetyltransferase (argJ), carbamoyl phosphate synthetase (carAB), but is not limited thereto.

In one embodiment, the protein involved in production of histidine among the amino acids may be at least one selected from proteins involved in histidine biosynthesis (histidine biosynthesis proteins). For example, the protein involved in histidine biosynthesis may be ATP phosphoribosyl transferase (hisG), phosphoribosyl-AMP cyclohydrolase (hisl), phosphoribosyl-ATP pyrophosphohydrolase (hisl), phosphoribosylformimino5-aminoimidazolecarboxamideribotideisomerase (hisA), amidotransferase (hisH), histidinol phosphate aminotransferase (hisC), histidinol phosphatase (hisB), histidinol dehydrogenase (hisD), but is not limited thereto.

In one embodiment, the protein involved in production of lysine among the amino acids may be at least one selected from proteins involved in lysine biosynthesis (lysine biosynthesis proteins). For example, the protein involved in lysine biosynthesis may be at least one selected from the group consisting of dihydrodipicolinate synthetase (dapA), aspartokinase III (lysC), dihydrodipicolinate reductase (dapB), diaminopimelate decarboxylase (lysA), diaminopimelate dehydrogenase (ddh), phosphoenolpyruvate carboxylase (ppc), aspartate semialdehyde dehydrogenase (asd), aspartate transaminase (aspC), diaminopimelate epimerase (dapF), tetrahydrodipicolinate succinylase (dapD), succinyl-diaminopimelate deacylase (dapE) and aspartase (aspA), but is not limited thereto.

In one embodiment, the protein involved in production of aspartic acid among the amino acids may be at least one selected from proteins involved in aspartic acid biosynthesis (aspartic acid biosynthesis proteins). For example, the protein involved in aspartic acid biosynthesis may be aspartate aminotransferase or the like, but is not limited thereto.

In one embodiment, the protein involved in production of glutamic acid among the amino acids may be at least one selected from proteins involved in glutamic acid biosynthesis (glutamic acid biosynthesis proteins). For example, the protein involved in glutamic acid biosynthesis may be at least one selected from the group consisting of glutamate dehydrogenase (gdhA), glutamine synthetase (glnA), glutamate synthetase (gltBD), isocitrate dehydrogenase (icdA), aconitate hydratase (acnA, acnB), citrate synthetase (gltA), methylcitrate synthetase (prpC), phosphoenolpyruvate carboxylase (ppc), pyruvate carboxylase (pyc), pyruvate dehydrogenase (aceEF, IpdA), pyruvate kinase (pykA, pykF), phosphoenolpyruvate synthetase (ppsA), enolase (eno), phosphoglyceromutase (pgmA, pgml), phosphoglyceric acid kinase (pgk), glyceraldehyde3-phosphate dehydrogenase (gapA), triosephosphate isomerase (tpiA), fructose bisphosphate aldolase (fbp), phosphofructokinase (pfkA, pfkB), glucose phosphate isomerase (pgi), 6-phosphogluconate dehydratase (edd), 2-kteo-3-deoxy-6-phosphogluconate aldolase (eda), transhydrogenase, or the like, but is not limited thereto.

In one embodiment, the protein involved in production of serine among the amino acids may be at least one selected from proteins involved in serine biosynthesis (serine biosynthesis proteins). For example, the protein involved in serine biosynthesis may be at least one selected from the group consisting of 3-phosphoglyceric acid dehydrogenase (serA), phosphoserine transaminase (serC), phosphoserine phosphatase (serB) or the like, but is not limited thereto.

In one embodiment, the protein involved in production of threonine among the amino acids may be at least one selected from proteins involved in threonine biosynthesis (threonine biosynthesis proteins). For example, the protein involved in threonine biosynthesis may be aspartokinase III (lysC), aspartate semialdehyde dehydrogenase (asd), aspartokinase I (thrA), homoserine kinase (thrB), threonine synthetase (thrC), aspartate aminotransferase, but is not limited thereto.

In one embodiment, the protein involved in production of asparagine among the amino acids may be at least one selected from proteins involved in asparagine biosynthesis (asparagine biosynthesis proteins). For example, the protein involved in asparagine biosynthesis may be transaminase, asparagine synthetase, or the like, but is not limited thereto.

In one embodiment, the protein involved in production of glutamine among the amino acids may be at least one selected from proteins involved in glutamine biosynthesis (glutamine biosynthesis proteins). For example, the protein involved in glutamine biosynthesis may be at least one selected from the group consisting of glutamate dehydrogenase (gdhA), glutamate synthetase (glnA), or the like, but is not limited thereto.

In one embodiment, the protein involved in production of cysteine among the amino acids may be at least one selected from proteins involved in cysteine biosynthesis (cysteine biosynthesis proteins). For example, the protein involved in cysteine biosynthesis may be at least one selected from the group consisting of serine acetyltransferase (cysE), 3-phosphoglycerate dehydrogenase (serA), or the like, but is not limited thereto.

In one embodiment, the protein involved in production of glycine among the amino acids may be at least one selected from proteins involved in glycine biosynthesis (glycine biosynthesis proteins). For example, the protein involved in glycine biosynthesis may be alanine-glyoxylate transaminase, or the like, but is not limited thereto.

In one embodiment, the protein involved in production of proline among the amino acids may be at least one selected from proteins involved in proline biosynthesis (proline biosynthesis proteins). For example, the protein involved in proline biosynthesis may be at least one selected from the group consisting of glutamate-5-kinase (proB), γ-glutamyl-phosphate reductase, pyrroline-5-carboxylate reductase (putA), and the like, but is not limited thereto. In one embodiment, the protein involved in production of alanine among the amino acids may be at least one selected from proteins involved in alanine biosynthesis (alanine biosynthesis proteins). For example, the protein involved in alanine biosynthesis may be alanine transaminase, or the like, but is not limited thereto.

In one embodiment, the protein involved in production of valine among the amino acids may be at least one selected from proteins involved in valine biosynthesis (valine biosynthesis proteins). For example, the protein involved in valine biosynthesis may be acetohydroxy acid isomeroreductase (IIvC), dihydroxy-acid dehydratase (IIvD), branched-chain amino acid aminotransferase (IIvE), but is not limited thereto.

In one embodiment, the protein involved in production of isoleucine among the amino acids may be at least one selected from proteins involved in isoleucine biosynthesis (isoleucine biosynthesis proteins). For example, the protein involved in isoleucine biosynthesis may be acetohydroxy acid synthetase (AHAS), acetohydroxy acid isomeroreductase, dihydroxy acid dehydratase, valine aminotransferase, or the like, but is not limited thereto.

In one embodiment, the protein involved in production of leucine among the amino acids may be at least one selected from proteins involved in leucine biosynthesis (leucine biosynthesis proteins). For example, the protein involved in leucine biosynthesis may be acetolactate synthase, acetohydroxy acid isomeroreductase, dihydroxy acid dehydratase, α-Isopropylmalic acid synthase, α-Isopropylmalate isomerase, leucine aminotransferase, or the like, but is not limited thereto.

In one embodiment, the protein involved in production of methionine among the amino acids may be at least one selected from proteins involved in methionine biosynthesis (methionine biosynthesis proteins). For example, the protein involved in methionine biosynthesis may be aspartic acid kinase, aspartic acid-semialdehyde dehydrogenase, homoserine dehydrogenase, homoserine O-succinyltransferase, cystathionine γ-generating enzyme, cystathionine β-lyase, methionine synthase, or the like, but is not limited thereto.

In one embodiment, the protein involved in production of phenylalanine among the amino acids may be at least one selected from proteins involved in phenylalanine biosynthesis (phenylalanine biosynthesis proteins). For example, the protein involved in phenylalanine biosynthesis may be chorismate mutase, prephenate aminotransferase, arogenate dehydratase, or the like, but is not limited thereto.

In one embodiment, the protein involved in production of tyrosine among the amino acids may be at least one selected from proteins involved in tyrosine biosynthesis (tyrosine biosynthesis proteins). For example, the protein involved in tyrosine biosynthesis may be chorismate mutase, prephenate aminotransferase, arogenate dehydrogenase, or the like, but is not limited thereto.

In one embodiment, the protein involved in production of tryptophan among the amino acids may be at least one selected from proteins involved in tryptophan biosynthesis (tryptophan biosynthesis proteins). For example, the protein involved in tryptophan biosynthesis may be anthranilate synthase, anthranilate phosphoribosyl transferase, anthranilate isomerase, imidazole glycerol phosphate synthase, tryptophan synthase, or the like, but is not limited thereto.

In one embodiment, the protein involved in production of O-acetyl homoserine among the amino acids may be at least one selected from proteins involved in O-acetyl homoserine biosynthesis. For example, the protein involved in O-acetyl homoserine biosynthesis may be homoserine O-acetyl transferase, or the like, but is not limited thereto.

In one embodiment, the protein involved in production of beta-alanine (β-alanine) among the amino acids may be at least one selected from proteins involved in beta-alanine biosynthesis. For example, the protein involved in beta-alanine biosynthesis may be propionate CoA ligase, medium chain acyl-CoA dehydrogenase, 3-hydroxypropionyl-CoA dehydratase, 3-hydroxypropionyl-CoA hydrolase, 3-hydroxypropionate dehydrogenase, beta alanine-pyruvate transaminase, or the like, but is not limited thereto.

The gene involved in production of the nucleic acid may be at least one or more genes selected from nucleic acid biosynthesis genes. For example, it may be at least one selected from the group consisting of amidophosphoribosyltransferase (purF), PRA-glycine ligase (purD), phosphoribosylaminoimidazolesuccinocarboxamide synthase (purC), bifunctional AICAR formyltransferase/IMP cyclohydrolase (purH), adenylosuccinate synthase (purA), adenylosuccinate lyase (purB), phosphoribosylaminoimidazole mutase (purE), phosphoribosylaminoimidazole carboxylase (purK), or the like, but is not limited thereto.

In one specific embodiment, the target gene may be at least one selected from the group consisting of pyc gene, metX gene, and ilvE gene.

According to one specific embodiment, the *pyc* gene encodes pyruvate carboxylase, and according to one specific embodiment, the polynucleotide and *pyc* gene are operably linked, and may be used for production of L-threonine. The pyc gene may be a gene derived from *Corynebacterium glutamicum.* In one embodiment, the pyc gene may be represented as Ncgl0659 and may comprise the nucleic acid sequence of SEQ ID NO: 21.

According to one specific embodiment, the *metX* gene encodes homoserine O-acetyltransferase, and according to one specific embodiment, the polynucleotide and *metX* gene are operably linked, and may be used for production for O-acetyl homoserine. The *metX* gene may be a gene derived from *Corynebacterium glutamicum.* In one embodiment, the *metX* gene may be represented as Ncgl0624 and may comprise the nucleic acid sequence of SEQ ID NO: 28.

According to one specific embodiment, the ilvE gene encodes branched-chain amino acid aminotransferase, and according to one specific embodiment, the polynucleotide and ilvE gene are operably linked, and may be used for production of valine. The ilvE gene may be a gene derived from *Corynebacterium glutamicum.* In one embodiment, the ilvE gene may be represented as Ncgl2123 and may comprise the nucleic acid sequence of SEQ ID NO: 32.

Another aspect provides an expression cassette comprising the polynucleotide and a gene encoding a target protein (hereinafter referred to as a target gene).

The target protein may be a protein (for example, enzyme) involved in production of a target product.

The target product may be at least one selected from the group consisting of amino acids, amino acid derivatives, nucleic acids, nucleic acid derivatives, vitamins, vitamin derivatives, sugars, sugar derivatives, fatty acids, fatty acid derivatives, proteins and other metabolites and the like.

The expression cassette may comprise the polynucleotide as a promoter.

In the present description, the term "expression cassette" may refer to a construct comprising at least a gene encoding a target protein and an expression regulatory sequence (for example, promoter), which is a polynucleotide fragment of the minimal unit capable of being expressed in a host cell.

The polynucleotide may be comprised at the 5' end or 3' end region of the gene encoding the target protein.

The polynucleotide may be operably linked to the gene encoding the target protein.

The target gene, target protein and target product are as described above.

Other aspect provides a vector, comprising the polynucleotide or the expression cassette.

The vector may be a recombinant vector (expression vector).

The vector may comprise the polynucleotide as a promoter.

According to one specific embodiment, the vector may comprise the polynucleotide having promoter activity and a gene encoding a target protein operably linked to the polynucleotide (hereinafter referred to as a target gene).

According to one specific embodiment, the target protein may be a fusion protein in which one protein (single protein), or 2 or more proteins are fused. When the target protein is a fusion protein comprising 2 or more proteins, the gene encoding target protein may be a fusion gene comprising genes encoding the 2 or more proteins, respectively.

According to one specific embodiment, when the gene encoding the target protein is a fusion gene comprising genes encoding 2 or more proteins, respectively, in the recombinant vector, it may be designed for all the genes comprised in the fusion gene to be under control of one polynucleotide (promoter), or for at least one of them to be under control of a separate polynucleotide. For example, the vector may comprise the polynucleotide having promoter activity and one gene or 2 or more (for example, 2, 3, 4, 5, 6, 7, 8, 9, or 10) genes operably linked to the polynucleotide, and when 2 or more genes are comprised, it may comprise one polynucleotide having promoter activity (that is, that the 2 or more genes are under control of one promoter), or comprise two or more (in this case, the polynucleotide may be comprised less than the number of the genes so that at least one of the 2 or more genes is under control of a separate promoter.

In the present description, the term "vector" collectively refers to any artificial DNA molecule that carries genetic material to enable the expression of a target gene within a suitable host cell, and may refer to a DNA construct comprising a suitable gene expression control sequence; and, optionally, a nucleic acid sequence of a target gene operably linked thereto. The "gene expression regulatory sequence" refers to elements that perform various regulatory functions in gene expression, and in one embodiment, it may comprise the polynucleotide having promoter activity provided in the present description, and may refer to a nucleic acid sequence capable of expressing a target gene operably linked thereto. Specifically, the gene expression regulatory sequence may comprise any operator sequence to regulate transcription, a sequence encoding an appropriate mRNA ribosome binding site, and/or a nucleic acid sequence that regulates termination of transcription and translation and the like, in addition to a promoter for carrying out transcription of the gene, but is not limited thereto. In addition, as a regulatory sequence suitable for prokaryotic organisms, a promoter and a ribosome binding site may be comprised, but is not limited thereto. The polynucleotide having promoter activity of the present application may be configured by a person skilled in the art to include sequences for regulating gene expression, as described above, as needed.

In the present description, "operably linked (operatively linked)" refers to that the polynucleotide having promoter activity is functionally linked to the nucleotide sequence of the target gene to initiate and/or mediate transcription of the target gene. Operative linking may be performed using a gene recombination technology known in the art, for example, site-specific DNA cleavage and/or linkage technologies, and the site-specific DNA cleavage and linkage may be performed using common lyase and/or ligase and the like, but is not limited thereto.

The vector is not particularly limited as long as it can be transformed into a host cell and/or expressed in the host cell. Examples of the vector may include plasmids, cosmids, viruses, and/or bacteriophages in a natural state or a recombinant state. For example, as the phage vector or cosmid vector, at least one selected from the group consisting of pWE15, M13, λLB3, λBL4, λfXII, λASHII, λAPII, λt10, λt11, Charon4A, Charon21A, and the like may be used, and as the plasmid vector, at least one selected from the group consisting of pBR-based, pUC-based, pBluescriptll-based, pGEM-based, pTZ-based, pCL-based, pET-based, and the like may be used.

In one embodiment, an endogenous promoter in chromosome of a host cell may be replaced with the polynucleotide having promoter activity provided in the present description. In this case, the vector may be a vector for inserting chromosome in a host cell comprising the polynucleotide having promoter activity, and for example, at least one selected from the group consisting of pECCG117, pDZ, pACYC177, pACYC184, pCL, pUC19, pBR322, pMW118, pCC1BAC, pCES208, pXMJ19 vectors and the like may be used, but is not limited thereto. In addition, insertion of the polynucleotide into chromosome may be performed by any method known in the art, for example, homologous recombination, genome editing by target-specific endonuclease (for example, RNA-guided endonuclease such as Cas9, Cpf1 and the like) and the like.

In one embodiment, when the vector or the polynucleotide comprised therein and/or target gene are inserted into chromosome, the vector may further comprise a selection marker for confirming chromosome insertion. The selection marker is for selecting cells transformed with the vector, i.e., for confirming whether the polynucleotide has been inserted. Markers that confer a selectable phenotype, such as drug (e.g., antibiotic) resistance, auxotrophy, resistance to a cytotoxic agents, or expression of a surface proteins, may be used. In an environment treated with a selective agent (drug, cytotoxic agent, etc.), only cells expressing the selection marker survive or show other phenotypes, so transformed cells may be selected.

Other aspect provides a recombinant cell comprising the polynucleotide or the recombinant vector in a host cell. In the present specification, the term "recombinant cell" may refer to a transformant in which the polynucleotide and/or the recombinant vector are introduced. The term "transformation" refers to introducing the polynucleotide having promoter activity or the recombinant vector into a suitable host cell so that a target gene under regulation of the polynucleotide (which may be comprised in the recombinant vector or may be an endogenous gene of the host cell) can be expressed in the host cell.

The host cell may include, without limitation, any cell in which the introduced polynucleotide can function as a promoter, and thereby the expression of a target gene (a gene included in a recombinant vector or an endogenous gene of the host cell) (operably linked thereto) under the regulation of the polynucleotide is enabled (or increased). In one embodiment, the host cell or recombinant cell may be one or more selected from the group consisting of microorganisms, plant cells, and animal cells. More specifically, the host cell may be a microorganism of the genus *Escherichia* or *Corynebacterium,* and even more specifically may be *Corynebacterium glutamicum,* but is not limited thereto.

The transformation method includes any method of introducing the polynucleotide or the recombinant vector into a host cell, and may be performed by selecting an appropriate standard technology known in the art depending on the host cell. For example, the transformation includes electroporation, lipofection, calcium phosphate (CaPO₄) precipitation, calcium chloride (CaCl₂) precipitation, microinjection, polyethylene glycol (PEG) method, DEAE-dextran method, cation liposome method, and lithium acetate-DMSO method and the like, but is not limited thereto.

In one embodiment, when the vector or the polynucleotide comprised therein and/or target gene are inserted into chromosome, the vector may further comprise a selection marker for confirming chromosome insertion. The selection marker is for selecting cells transformed with the vector, i.e., for confirming whether the polynucleotide has been inserted. Markers that confer a selectable phenotype, such as drug (e.g., antibiotic) resistance, auxotrophy, resistance to a cytotoxic agents, or expression of a surface proteins, may be used. In an environment treated with a selective agent (drug, cytotoxic agent, etc.), only cells expressing the selection marker survive or show other phenotypes, so transformed cells may be selected.

Other aspect provides a microorganism comprising at least one selected from the group consisting of the polynucleotide, the expression cassette, and the vector.

In one embodiment, the microorganism may comprise the polynucleotide and the expression cassette.

The microorganism (or strain, or recombinant cell) of the present application may be a microorganism which has target product production ability, or has improved (or increased) target product production ability.

The microorganism of the present application may be a microorganism in which at least one selected from the group consisting of the polynucleotide, the expression cassette, and the vector is introduced into a microorganism naturally lacking a production ability of a target product or a microorganism already having a production ability of a target product, thereby conferring or improving the production ability of the target product, but is not limited thereto. The microorganism in which the production ability of the target product is conferred or improved may be a microorganism into which at least one selected from the group consisting of the polynucleotide, the expression cassette, and the vector is introduced.

The fact that the microorganism has improved production ability of the target product or has production ability of the target product may mean that the production ability of the target product is improved compared to an unmodified microorganism, a cell before recombination, a parent strain, and/or a wild-type strain; or that the production ability of the target product is conferred, unlike an unmodified microorganism, a cell before recombination, a parent strain, and/or a wild-type strain that lacks the production ability of the target product.

The microorganism in which at least one selected from the group consisting of the polynucleotide, the expression cassette and the vector is introduced may have improved target product production ability, compared to a microorganism before introduced or a microorganism before intensified, that is, a homogenous unmodified microorganism. Herein, "unmodified microorganism" does not exclude a strain which comprises a mutation that can occur naturally in the microorganism, and may mean a wild-type strain or a natural strain itself, or a strain before traits are changed by genetic mutation due to a natural or artificial factor. For example, the unmodified microorganism may refer to a strain in which at least one selected from the group consisting of the polynucleotide, the expression cassette and the vector is not introduced, or before it is introduced. The "unmodified microorganism" may be interchangeably used with "strain before modified", "microorganism before modified", "non-mutated strain", "unmodified strain", "non-mutated strain", "non-mutated microorganism" or "reference microorganism". At least one selected from the group consisting of the polynucleotide, the expression cassette and the vector are as described above.

In the present specification, the term "recombinant cell" may refer to a transformant in which the polynucleotide and/or the recombinant vector are introduced.

The term "transformation" refers to introducing the polynucleotide having promoter activity or the recombinant vector into a suitable host cell so that a target gene under regulation of the polynucleotide (which may be comprised in the recombinant vector or may be an endogenous gene of the host cell) can be expressed in the host cell.

The host cell may include, without limitation, any cell in which the introduced polynucleotide can function as a promoter, and thereby the expression of a target gene (a gene included in a recombinant vector or an endogenous gene of the host cell) (operably linked thereto) under the regulation of the polynucleotide is enabled (or increased).

**In** one specific embodiment, the host cell or recombinant cell may be at least one selected from the group consisting of various microorganisms, plant cells, animal cells, and the like, and for example, may be a microorganism of the genus *Escherichia* (microorganism of *Escherichia* sp.), microorganism of the genus *Corynebacterium* (microorganism of *Corynebacterium* sp.), or microorganism of the genus *Bacillus* (microorganism of *Bacillus* sp.), or the like, and more specifically, it may be *Corynebacterium glutamicum, Escherichia coli* or *Bacillus subtilis,* but is not limited thereto.

**In** one embodiment, the microorganism may be a microorganism of the genus *Corynebacterium,* a microorganism of the genus *Escherichia,* and/or a microorganism of the genus *Bacillus.*

The microorganism of the genus *Corynebacterium* may be at least one selected from the group consisting of *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris, Corynebacterium acetoacidophilum, Corynebacterium acetoglutamicum, Corynebacterium alkanolyticum, Corynebacterium lilium, Corynebacterium melassecola, Corynebacterium thermoaminogenes, Corynebacterium herculis* and *Corynebacterium flavescens,* but is not limited thereto.

The microorganism of the genus *Bacillus* may be at least one selected from the group consisting of *Bacillus subtilis, Bacillus amyloliquefaciens, Bacillus methylotrophicus, Bacillus licheniformis, Bacillus velezensis, Bacillus sonorensis* and *Bacillus valismortis,* but is not limited thereto.

The microorganism of the genus *Escherichia* may be *Escherichia coli,* but is not limited thereto.

In one embodiment, a microorganism into which at least one selected from the group consisting of the polynucleotide, the expression cassette and the vector is introduced may be one in which the production ability of the target product is newly conferred, or increased by about 2% or more, about 3% or more, about 4% or more, about 5% or more, about 6% or more, about 7% or more, about 8% or more, about 9% or more, about 10% or more, about 11% or more, about 12% or more, about 13% or more, about 14% or more, about 15% or more, about 16% or more, about 20% or more, about 25% or more, about 30% or more, about 40% or more, about 50% or more, about 60% or more, about 70% or more, about 80% or more, about 90% or more, about 100% or more, about 150% or more, about 200% or more, about 250% or more, about 300% or more, about 400% or more, about 500% or more, about 600% or more, about 700% or more, about 800% or more, about 900% or more, about 1,000% or more, about 1,500% or more, about 2,000% or more, about 2,500% or more, or about 3,000% or more, compared to a parent strain before mutation, unmodified microorganism, parent strain, or wild-type microorganism, but is not limited thereto.

In another embodiment, the microorganism with the improved production ability of target product may have a production ability of the target product of 1.02-fold or more, about 1.03-fold or more, about 1.04-fold or more, about 1.05-fold or more, about 1.06-fold or more, about 1.07-fold or more, about 1.08-fold or more, about 1.09-fold or more, about 1.1-fold or more, about 1.11-fold or more, about 1.12-fold or more, about 1.13-fold or more, about 1.14-fold or more, about 1.15-fold or more, about 1.16-fold or more, about 1.2-fold or more, about 1.3-fold or more, about 1.4-fold or more, about 1.5-fold or more, about 1.6-fold or more, about 1.7-fold or more, about 1.8-fold or more, about 1.9-fold or more, about 2-fold or more, about 2.5-fold or more, about 3-fold or more, about 4-fold or more, about 5-fold or more, about 6-fold or more, about 7-fold or more, about 8-fold or more, about 9-fold or more, about 10-fold or more, about 15-fold or more, about 20-fold or more, about 25-fold or more, or about 30-fold or more (the upper limit is not particularly limited, and for example, it may be about 1,000-fold or less) compared to the parent strain before mutation, and unmodified microorganism, but is not limited thereto.

In another embodiment, the microorganism with improved production ability of the target product may have a production ability of the target product of approximately 0.1 g/L or more, approximately 0.2 g/L or more, approximately 0.3 g/L or more, approximately 0.4 g/L or more, approximately 0.5 g/L or more, approximately 0.6 g/L or more, approximately 0.7 g/L or more, approximately 0.8 g/L or more, approximately 0.9 g/L or more, approximately 1 g/L or more, approximately 1.1 g/L or more, approximately 1.2 g/L or more, approximately 1.3 g/L or more, approximately 1.4 g/L or more, approximately 1.5 g/L or more, approximately 1.6 g/L or more, approximately 1.7 g/L or more, approximately 1.8 g/L or more, approximately 1.9 g/L or more, approximately 2.0 g/L or more, approximately 2.5 g/L or more, approximately 3 g/L or more, approximately 3.5 g/L or more, approximately 4 g/L or more, approximately 4.5 g/L or more, approximately 5 g/L or more, approximately 5.5 g/L or more, approximately 6 g/L or more, approximately 7 g/L or more, approximately 8 g/L or more, approximately 9 g/L or more, approximately 10 g/L or more, approximately 15 g/L or more, approximately 20 g/L or more, approximately 25 g/L or more, or approximately 30 g/L or more (the upper limit is not particularly limited and may be, for example, approximately 100 g/L or less) compared to a parent strain before mutation or an unmodified microorganism, but is not limited thereto.

The term, "about" is a range including all of ±0.5, ±0.4, ±0.3, ±0.2, ±0.1 and the like, and includes all numerical values in the equivalent or similar range to the numerical value after the term, about.

The transformation method includes any method of introducing the polynucleotide or the recombinant vector into a host cell, and may be performed by selecting an appropriate standard technology known in the art depending on the host cell. For example, the transformation includes electroporation, lipofection, calcium phosphate (CaPO₄) precipitation, calcium chloride (CaCl₂) precipitation, microinjection, polyethylene glycol (PEG) method, DEAE-dextran method, cation liposome method, and lithium acetate-DMSO method and the like, but is not limited thereto.

Other aspect provides a composition for producing a target product comprising the microorganism, a medium in which the microorganism is cultured, or a combination thereof.

The composition may further comprise any appropriate excipient commonly used in the composition for producing a target product, and this excipient, may be, for example, a preservative, a wetting agent, a dispersant, a buffer, a stabilizer, or a tonicifying agent, but is not limited thereto.

Other aspect provides a use of the microorganism, a medium in which the microorganism is cultured, or a combination thereof for producing a target product.

Other aspect provides a use of the microorganism for using in the manufacture of a composition for producing a target product.

Other aspect provides a method for producing a target product, comprising culturing the microorganism in a medium.

The method may further comprise recovering the target product from the medium or the microorganism resulting from the culturing.

The medium in which the microorganism is cultured may comprise or not comprise the microorganism.

The microorganism may comprise at least one selected from the group consisting of the polynucleotide provided herein, an expression cassette comprising the polynucleotide and a target gene, and a vector comprising the expression cassette, as described above.

In the present description, "culture" refers to growing cells under an artificially controlled environmental condition. The method for producing a target product provided in the present description may be performed by appropriately selected means among all methods widely known in the art. For example, the culture may be continuously culturing in a batch process, a fed batch or repeated fed batch process, but is not limited thereto. The medium used for culturing may satisfy growth requirements of cells subjected to culturing in an appropriate method.

Herein, "medium" refers to a substance in which nutrients required for culturing the microorganism, for example, Corynebacterium glutamicum strain, are mixed as major components, and nutrients and growth factors and the like including water essential for survival and development are supplied. Specifically, for the medium used for the culturing of the microorganism of the present application and other culture condition, any one may be used without particular limitations, as long as it is a medium used for culturing a common microorganism, but the microorganism of the present application may be cultured in a common medium containing suitable carbon sources, nitrogen sources, phosphorus sources, inorganic compounds, amino acids, and/or vitamins, while adjusting the temperature, pH and the like under an aerobic condition.

Specifically, the culture medium for the microorganism of the present application, for example, the microorganism of the genus *Corynebacterium* may be found in the document ["Manual of Methods for General Bacteriology" by the American Society for Bacteriology (Washington D.Corynebacterium, USA, 1981)].

Sugar sources which may be used for the culturing or comprised in the medium may comprise at least one selected from the group consisting of sugars and carbohydrates such as glucose, saccharose, lactose, fructose, maltose, starch, cellulose and the like; oils and fats such as soybean oil, sunflower oil, castor oil, coconut oil and the like; fatty acids such as palmitic acid, stearic acid, and linoleic acid; alcohols such as glycerol, ethanol, and the like; organic acids such as acetic acid, and the like, but are not limited thereto. Nitrogen sources which may be used for the culturing or comprised in the medium, may include at least one selected from the group consisting of organic nitrogen sources such as peptone, yeast extracts, meat juice, malt extracts, corn steep liquor, soybean meal, urea and the like, and inorganic nitrogen sources such as ammonium sulfate, ammonium chloride, ammonium phosphate, ammonium carbonate, ammonium nitrate and the like, but are not limited thereto. Phosphorus sources which may be used for the culturing or comprised in the medium, may comprise at least one selected from the group consisting of potassium salts of phosphate such as potassium dihydrogen phosphate, dipotassium hydrogen phosphate and the like and sodium salts corresponding thereto, and the like, but are not limited thereto. In addition thereto, the medium may contain metal salts such as magnesium sulfate or iron sulfate required for growth. Furthermore, in addition thereto, the culturing or medium may further use or comprise at least one selected from essential growth substances such as amino acids and vitamins. Moreover, the medium may comprise an appropriate precursor of the target product as a raw material. The raw materials described above may be added to the culture in batches and/or continuously in the culturing process.

During the culturing of cells, using base compounds such as sodium hydroxide, potassium hydroxide and ammonia, and/or acid compounds such as phosphate or sulfate in an appropriate manner, the pH of the culture may be adjusted. In addition, during the culturing, using an antifoaming agent such as fatty acid polyglycol ester and the like, foam generation may be inhibited. Furthermore, in order to maintain the aerobic condition, oxygen or oxygen-containing gas (e.g., air) may be injected into the culture. The temperature of the medium and/or culture may be commonly 20°C to 45°C, or 25°C to 40°C.

The culturing time may continue until the production of the target product reaches the desired amount, and for example, it may be about 10 to about 160 hours, but is not limited thereto.

The isolating or recovering the target substance from the cultured microorganism or culture medium may be performed using an appropriate method known in the art depending on the culturing method. For example, a method such as centrifugation, filtration, extraction, spray, drying, evaporation, precipitation, crystallization, electrophoresis, fractional dissolution (for example, ammonium sulfate precipitation), and/or chromatography (for example, ion exchange, affinity, hydrophobic and size exclusion) and the like, but is not limited thereto. The culturing medium refers to a medium in which recombinant cells are cultured.

According to one specific embodiment, the isolating or recovering the target product may be isolating supernatant obtained by low-speed centrifuging the culture to remove biomass through ion exchange chromatography.

The method for producing the target product may further comprise a process of purifying the target product.

The recovering the target product from the culture (medium) may be performed by an isolation method by common means known in the art. As the common means which may be used for isolating of the target product, centrifugation, filtration, chromatography and/or crystallization methods and the like may be exemplified. In one embodiment, the target product may be isolated by performing ion exchange chromatography for the supernatant obtained by low-speed centrifuging the culture to remove biomass, but is not limited thereto. The recovering may further comprise a purification process.

### [ADVANTAGEOUS EFFECTS]

The polynucleotide according to one specific embodiment has promoter activity, and is introduced into a microorganism, and thereby, can increase expression and activity of a gene operably linked thereto, and can be usefully used for efficiently producing a target product affected by the polynucleotide and gene.

### [MODE FOR INVENTION]

The present invention will be described in more detail with reference to the following Examples; however, the scope of the present invention is not intended to be limited by these Examples.

### Example 1. Construction of a recombinant vector comprising an improved promoter and preparation of a transformed strain

### Example 1-1. Construction of a recombinant vector comprising an improved o2 promoter sequence

A mutant promoter was prepared based on a conventional o2 promoter known to exhibit strong activity (U.S. Patent No. US 10,273,491 B2).

Using the o2 promoter consisting of the nucleotide sequence of SEQ ID NO: 1 (hereinafter referred to as "Po2") as a template, random mutagenesis was induced using a Diversify PCR Random Mutagenesis Kit in accordance with the manufacturer's manual to obtain two promoters having different sequences.

Specifically, among the two promoters obtained, a promoter consisting of a nucleic acid sequence (SEQ ID NO: 39) in which the 21st nucleotide A of the nucleic acid sequence represented by SEQ ID NO: 1 was substituted with C was designated as "Po2.1", and a promoter consisting of a nucleic acid sequence (SEQ ID NO: 40) in which the 19th nucleotide G of the nucleic acid sequence represented by SEQ ID NO: 1 was substituted with A was designated as "Po2.2".

In addition, two promoters were obtained in which additional sequences were added to the 3' terminus of the promoter of SEQ ID NO: 1.

Specifically, a promoter consisting of a nucleic acid sequence (SEQ ID NO: 41) in which the nucleotide sequence "CAT" was added to the 3' terminus of SEQ ID NO: 1 was designated as "Po2.3", and a promoter consisting of a nucleic acid sequence (SEQ ID NO: 42) in which the nucleotide sequence "ACTAGT" was added to the 3' terminus of SEQ ID NO: 1 was designated as "Po2.4".

The nucleic acid sequences of Po2, Po2.1, Po2.2, Po2.3, and Po2.4 are shown in Table 1 below, and substituted or added sequences are underlined.

**[Table 1]**

| Promoter (SEQ ID NO.) | Sequence (5'→3') |
|---|---|
| Po2 (SEQ ID NO. 1) | caataatcgtgaattttggcagcaacagaattatgtggtataatggaaacgtg caaaagcatagattattggaggagatcaaaaca |
| Po2.1 (SEQ ID NO. 39) | caataatcgtgaattttggccgcaacagaattatgtggtataatggaaacgtg caaaagcatagattattggaggagatcaaaaca |
| Po2.2 (SEQ ID NO. 40) | caataatcgtgaattttgacagcaacagaattatgtggtataatggaaacgtg caaaagcatagattattggaggagatcaaaaca |
| Po2.3 (SEQ ID NO. 41) | caataatcgtgaattttggcagcaacagaattatgtggtataatggaaacgtg caaaagcatagattattggaggagatcaaaacacat |
| Po2.4 (SEQ ID NO. 42) | caataatcgtgaattttggcagcaacagaattatgtggtataatggaaacgtg caaaagcatagattattggaggagatcaaaacaactagt |

### Example 1-1-1. Construction of pCES_Po2.1_gfp, pCES_Po2.2_gfp, pCES_Po2.3_gfp and pCES_Po2.4_gfp vectors

To construct recombinant GFP expression vectors regulated by Po2.1, Po2.2, Po2.3, or Po2.4, PCR was performed using Po2.1 and Po2.2 as templates with primers of SEQ ID NOs: 2 and 3, respectively; using Po2.3 as a template with primers of SEQ ID NOs: 2 and 4; and using Po2.4 as a template with primers of SEQ ID NOs: 2 and 5. PCR was carried out by denaturation at 94 °C for 5 minutes, followed by 30 cycles of denaturation at 94 °C for 30 seconds, annealing at 55 °C for 30 seconds, and extension at 72 °C for 1 minute, and a final extension at 72 °C for 5 minutes, thereby obtaining PCR products corresponding to Po2.1, Po2.2, Po2.3, and Po2.4.

In addition, the ORF (open reading frame) of the GFP gene was amplified using the pGFPuv vector (Clontech, USA) as a template with primers of SEQ ID NOs: 6 and 7, primers of SEQ ID NOs: 8 and 7, and primers of SEQ ID NOs: 9 and 7, respectively, thereby obtaining gene fragments each comprising the GFP ORF. PCR was performed under the same conditions as described above.

Further, PCR was performed using the *Escherichia coli-Corynebacterium* shuttle vector pCES208 (J. Microbiol. Biotechnol., 18:639-647, 2008) as a template with primers of SEQ ID NOs: 10 and 11 to prepare a vector for introducing the mutant promoter sequences Po2.1, Po2.2, Po2.3, and Po2.4 and the GFP expression sequence. PCR was carried out by denaturation at 94 °C for 5 minutes, followed by 30 cycles of denaturation at 94 °C for 30 seconds, annealing at 55 °C for 30 seconds, and extension at 72 °C for 5 minutes, and a final extension at 72 °C for 5 minutes.

Thereafter, the Po2.1 fragment, Po2.2 fragment, Po2.3 fragment, Po2.4 fragment, the ORF fragment of the GFP gene, and the pCES208 vector obtained by the above PCR method were assembled using an In-Fusion enzyme (BD In-Fusion kit) to construct recombinant vectors in which Po2.1, Po2.2, Po2.3, and Po2.4 were respectively linked to the ORF of the GFP gene. The resulting vectors were designated as "pCES_Po2.1_gfp", "pCES_Po2.2_gfp", "pCES_Po2.3_gfp", and "pCES_Po2.4_gfp", respectively.

### Example 1-1-2. Construction of pHT_Po2.1_gfp and pHT_Po2.2_gfp vectors

To construct recombinant vectors for Bacillus expression comprising Po2.1 or Po2.2, PCR was performed using Po2.1 and Po2.2 as templates with primers of SEQ ID NOs: 12 and 13, respectively. PCR was carried out by denaturation at 94 °C for 5 minutes, followed by 30 cycles of denaturation at 94 °C for 30 seconds, annealing at 55 °C for 30 seconds, and extension at 72 °C for 1 minute, and a final extension at 72 °C for 1 minute, thereby obtaining PCR products corresponding to Po2.1 and Po2.2.

In addition, PCR was performed using the pGFPuv vector as a template with primers of SEQ ID NOs: 14 and 15 to obtain gene fragments each comprising the GFP ORF. PCR was performed under the same conditions as described above.

Further, PCR was performed using the pHT43 vector (Mobitec GmbH) as a template with primers of SEQ ID NOs: 16 and 17 to prepare a vector for introducing the improved promoter sequences Po2.1 and Po2.2. PCR was carried out by denaturation at 94 °C for 5 minutes, followed by 30 cycles of denaturation at 94 °C for 30 seconds, annealing at 55 °C for 30 seconds, and extension at 72 °C for 5 minutes, and a final extension at 72 °C for 5 minutes.

Thereafter, the Po2.1 fragment, Po2.2 fragment, and the ORF fragment of the GFP gene were operably linked to the pHT43 vector obtained by the above PCR method using an In-Fusion enzyme to construct recombinant vectors in which Po2.1 or Po2.2 was respectively linked to the ORF of the GFP gene. These resulting vectors were designated as "pHT_Po2.1_gfp" and "pHT_Po2.2_gfp", respectively.

### Example 1-1-3. Construction of pCES_Po2_gfp, pHT_Po2_gfp, and pHT_P43_gfp vectors comprising control promoters

As a control for confirming the activity of the improved Po2 promoter variants, a recombinant vector in which an unmodified Po2 promoter (U.S. Patent No. US 10,273,491 B2) was linked to GFP was used. PCR was performed using Po2 as a template with primers of SEQ ID NOs: 2 and 3, and in the same manner as described in Example 1-1-1, the Po2 fragment and the ORF fragment of the GFP gene were operably linked into the pCES208 vector using a BD In-Fusion kit to construct a recombinant vector in which Po2 was linked to GFP. The resulting vector was designated as "pCES_Po2_gfp".

In addition, as a control for confirming the activity of the improved Po2 promoter variants in *Bacillus subtilis,* a recombinant vector in which an unmodified Po2 promoter (U.S. Patent No. US 10,273,491 B2) was linked to GFP was used. PCR was performed using Po2 as a template with primers of SEQ ID NOs: 12 and 13, and in the same manner as described in Example 1-1-2, the Po2 fragment and the ORF fragment of the GFP gene were operably linked into the pHT43 vector using a BD In-Fusion kit to construct a recombinant vector in which Po2 was linked to GFP. The resulting vector was designated as "pHT_Po2_gfp".

As another control for confirming the activity of the improved Po2 promoter variants in *Bacillus subtilis,* a conventionally known strong promoter P43 (Wang and Doi, Journal of Biological Chemistry, 1984) was used as a template, and PCR was performed using primers of SEQ ID NOs: 18 and 19 to obtain the P43 fragment. In addition, PCR was performed using the pGFPuv vector as a template with primers of SEQ ID NOs: 20 and 15 to obtain a gene fragment comprising the GFP ORF. Thereafter, in the same manner as described above, the P43 fragment and the ORF fragment of the GFP gene were operably linked into the pHT43 vector using a BD In-Fusion kit to construct a recombinant vector in which P43 was linked to GFP. The resulting vector was designated as "pHT_P43_gfp".

The primers of SEQ ID NOs: 2 to 20 used above are shown in Table 2 below.

**[Table 2]**

| SEQ IN NO. | Sequence (5' → 3') |
|---|---|
| SEQ ID NO. 2 | CTCTAGAACTAGTGGATCCCCCcaataatcgtgaattttgg |
| SEQ ID NO. 3 | AAAGTTCTTCTCCTTTACTCATtattttgatctcctccaataat |
| SEQ ID NO. 4 | AAAGTTCTTCTCCTTTACTCATatgtgttttgatctcctccaataat |
| SEQ ID NO. 5 | AAAGTTCTTCTCCTTTACTCATactatgtgttttgatctcctccaataat |
| SEQ ID NO. 6 | attattggaggagatcaaaacaATGAGTAAAGGAGAAGAACTTT |
| SEQ ID NO. 7 | TGATATCGAATTCCTGCAGCCCTTATTTGTAGAGCTCATCCA TG |
| SEQ ID NO. 8 | attattggaggagatcaaaacacatATGAGTAAAGGAGAAGAACTTT |
| SEQ ID NO. 9 | attattggaggagatcaaaacaactagtATGAGTAAAGGAGAAGAACTTT |
| SEQ ID NO. 10 | GGGCTGCAGGAATTCGATATCAAGC |
| SEQ ID NO. 11 | GGGGGATCCACTAGTTCTAGAGCGG |
| SEQ ID NO. 12 | GAAAAATTTTTTATCTTATCcaataatcgtgaattttgg |
| SEQ ID NO. 13 | AGTTCTTCTCCTTTACTCATtgttttgatctcctccaata |
| SEQ ID NO. 14 | tattggaggagatcaaaacaATGAGTAAAGGAGAAGAACT |
| SEQ ID NO. 15 | AGGCGGGCTGCCCCGGGGACTTATTTGTAGAGCTCATCCA |
| SEQ ID NO. 16 | GTCCCCGGGGCAGCCCGCCT |
| SEQ ID NO. 17 | GATAAGATAAAAAATTTTTC |
| SEQ ID NO. 18 | AATGTAGTGAGGTGGATGCAATGAGTAAAGGAGAAGAACT |
| SEQ ID NO. 19 | GAAAAATTTTTTATCTTATCATGAGTAAAGGAGAAGAACT |
| SEQ ID NO. 20 | AATGTAGTGAGGTGGATGCAATGAGTAAAGGAGAAGAACT |

### Example 1-2. Preparation of transformed strains

The recombinant vectors pCES_Po2.1_gfp, pCES_Po2.2_gfp, pCES_Po2.3_gfp, and pCES_Po2.4_gfp constructed in Example 1-1, and pCES_Po2_gfp comprising the unmodified Po2 promoter were each introduced into *Corynebacterium glutamicum* ATCC13032 by electroporation (Appl. Microbiol. Biotechnol. (1999) 52:541-545). The transformed strains were selected on BHIS agar plates (brain heart infusion 37 g/L, sorbitol 91 g/L, agar 2%) containing kanamycin (25 mg/L), and were designated as "ATCC13032/pCES_Po2.1_gfp", "ATCC13032/pCES_Po2.2_gfp", "ATCC13032/pCES_Po2.3_gfp", "ATCC13032/pCES_Po2.4_gfp", and "ATCC13032/pCES_Po2_gfp", respectively.

In addition, the recombinant vectors pCES_Po2.1_gfp, pCES_Po2.2_gfp, pCES_Po2.3_gfp, and pCES_Po2.4_gfp constructed in Example 1-1, and pCES_Po2_gfp comprising the unmodified Po2 promoter were each introduced into *Escherichia coli* DH5α by heat shock. The transformed strains were selected on Luria-Bertani (LB) agar plates containing kanamycin (25 mg/L), and were designated as "DH5α/pCES_Po2.1_gfp", "DH5α/pCES_Po2.2_gfp", "DH5α/pCES_Po2.3_gfp", "DH5α/pCES_Po2.4_gfp", and "DH5α/pCES_Po2_gfp", respectively.

Further, the recombinant vectors pHT_Po2.1_gfp and pHT_Po2.2_gfp constructed in Example 1-1, and pHT_Po2_gfp and pHT_P43_gfp comprising the unmodified Po2 promoter were each introduced into *Bacillus subtilis* ATCC23857 by electroporation (Eppendorf Protocol No. 4308 915 504). The transformed strains were selected on a selection medium containing kanamycin (50 mg/L), and were designated as "ATCC23857/pHT_Po2.1_gfp", "ATCC23857/pHT_Po2.2_gfp", "ATCC23857/pHT", "ATCC23857/pHT_Po2_gfp", and "ATCC23857/pHT_P43_gfp", respectively.

### Example 1-3. Evaluation of activity of improved promoters

### Example 1-3-1. Evaluation of expression-inducing activity of Po2.1, Po2.2, Po2.3, and Po2.4 in Corynebacterium glutamicum

To evaluate the activity of the Po2 promoter variants, the transformed *Corynebacterium glutamicum* strains obtained in Example 1-2, namely "ATCC13032/pCES_Po2.1_gfp", "ATCC13032/pCES_Po2.2_gfp", "ATCC13032/pCES_Po2.3_gfp", "ATCC13032/pCES_Po2.4_gfp", and "ATCC13032/pCES_Po2_gfp", were cultured as described below, and GFP activity was evaluated.

Specifically, the transformed *Corynebacterium glutamicum* strains were each inoculated at a 1/20 ratio (v/v) into 250 mL corner-baffled flasks containing 25 mL of seed medium (pH 7.2), and shake-cultured at 30 °C and 200 rpm until the mid-log phase (OD600 = 10.0). The composition of the seed medium is shown in Table 3 below. Cells were harvested from the culture by centrifugation (5,000 rpm, 15 minutes), washed twice with 0.1% Tris-HCl buffer (pH 8.0), and resuspended in the same buffer to a turbidity of approximately 160 at 610 nm. Glass beads (1.25 g per 1.5 mL of suspension) were added, and the cells were disrupted for 6 minutes using a bead beater. The supernatant was collected by centrifugation (15,000 rpm, 20 minutes), and protein concentration was quantified using the Bradford method (Bradford, M.M., 1976. Anal. Biochem. 72:248-254). For equal amounts of cell extract, irradiation with excitation light at 488 nm and measurement of emission at 511 nm were performed using an LS-50B spectrophotometer (Perkin-Elmer) according to the method of Laure Gory et al., thereby evaluating the expression level of the GFP gene. The results are shown in Table 4 below.

**[Table 3]**

| Medium type | Composition |
|---|---|
| Seed medium (pH 7.2) | glucose 20g, ammonium sulfate 5g, yeast extract 5g, urea 1.5g, KH₂PO₄ 4g, K₂HPO₄ 8g, MgSO₄7H₂O 0.5g, biotin 150µg, thiamine hydrochloride 1.5mg, calcium pantothenate 3mg, nicotinamide 3mg (per 1 L of distilled water), pH 7.2 |

**[Table 4]**

| Strain | Fluorescence intensity | Relative fluorescence intensity |
|---|---|---|
| ATCC13032/pCES_Po2_gfp | 2315 | 100% |
| ATCC13032/pCES_Po2.1_gfp | 2895 | 120% |
| ATCC13032/pCES_Po2.2_gfp | 3627 | 157% |
| ATCC13032/pCES_Po2.3_gfp | 2330 | 101% |
| ATCC13032/pCES_Po2.4_gfp | 2259 | 98% |

As shown in Table 4, the four Po2 variants exhibited promoter activity in *Corynebacterium glutamicum,* and among them, the Po2.1 and Po2.2 promoters showed higher fluorescence intensity than ATCC13032/pCES_Po2_gfp using the conventional strong promoter Po2.

### Example 1-3-2. Evaluation of expression-inducing activity of Po2.1, Po2.2, Po2.3, and Po2.4 in Escherichia coli

To evaluate the activity of the Po2 promoter variants in *Escherichia coli,* the transformed strains obtained in Example 1-2, namely "DH5α/pCES_Po2.1_gfp", "DH5α/pCES_Po2.2_gfp", "DH5α/pCES_Po2.3_gfp", "DH5α/pCES_Po2.4_gfp", and "DH5α/pCES_Po2_gfp", were cultured as described below, and GFP activity was evaluated.

Specifically, the transformed *Escherichia coli* strains were each inoculated at a 1/20 ratio (v/v) into 250 mL corner-baffled flasks containing 25 mL of LB medium supplemented with kanamycin, and shake-cultured at 37 °C and 200 rpm until the mid-log phase (OD600 = 3.0). Cells were harvested from the culture by centrifugation (5,000 rpm, 15 minutes), washed twice with 0.1% Tris-HCl buffer (pH 8.0), resuspended in the same buffer, and disrupted by sonication. The supernatant was collected by centrifugation (15,000 rpm, 20 minutes), and protein concentration was quantified using the Bradford method. For equal amounts of cell extract, excitation at 488 nm and emission at 511 nm were measured using an LS-50B spectrophotometer (Perkin-Elmer) according to the method of Laure Gory et al., thereby evaluating the expression level of the GFP gene. The results are shown in Table 5 below. For equal amounts of cell extract, irradiation with excitation light at 488 nm and measurement of emission at 511 nm were performed using an LS-50B spectrophotometer (Perkin-Elmer) according to the method of Laure Gory et al., thereby evaluating the expression level of the GFP gene. The results are shown in Table 5 below.

**[Table 5]**

| Strain | Fluorescence intensity | Relative fluorescence intensity |
|---|---|---|
| DH5α/pCES_Po2_gfp | 260 | 100% |
| DH5α/pCES_Po2.1_gfp | 308 | 118% |
| DH5α/pCES_Po2.2_gfp | 301 | 116% |
| DH5α/pCES_Po2.3_gfp | 270 | 103% |
| DH5α/pCES_Po2.4_gfp | 250 | 96% |

As shown in Table 5, the four Po2 variants exhibited promoter activity in *Escherichia coli,* and among them, the Po2.1 and Po2.2 promoters showed higher fluorescence intensity than Po2, which is known as a strong conventional promoter.

### Example 1-3-1. Evaluation of expression-inducing activity of Po2.1 and Po2.2 in Bacillus subtilis

To evaluate the activity of the o2.1 and o2.2 promoters in Bacillus subtilis, the transformed Bacillus subtilis strains obtained in Example 1-2, namely ATCC23857/pHT_P43_gfp, ATCC23857/pHT_Po2.1_gfp, ATCC23857/pHT_Po2.2_gfp, and ATCC23857/pHT_Po2_gfp, were cultured by the following method, and GFP activity was measured.

Specifically, the transformed *Bacillus subtilis* strains were each inoculated at a ratio of 1/20 into 250 mL corner-baffled flasks containing 25 mL of seed medium, and shake-cultured at 37 °C and 200 rpm until the mid-log phase (OD600 = 10.0). The composition of the seed medium is described in Table 6 below. Cells were harvested from the culture by centrifugation (5,000 rpm, 15 minutes), washed twice with 0.1% Tris-HCl buffer (pH 8.0), and resuspended in the same buffer to a turbidity of approximately 160 at 610 nm. Glass beads (1.25 g per 1.5 mL of suspension) were added, and the cells were disrupted for 6 minutes using a bead beater. The supernatant was collected by centrifugation (15,000 rpm, 20 minutes), and protein concentration was quantified using the Bradford method (Bradford, M.M., 1976. Anal. Biochem. 72:248-254). For equal amounts of cell extract, irradiation with excitation light at 488 nm and measurement of emission at 511 nm were performed using an LS-50B spectrophotometer (Perkin-Elmer) according to the method of Laure Gory et al., thereby evaluating the expression level of the GFP gene. The results are shown in Table 7 below.

**[Table 6]**

| Medium type | Composition |
|---|---|
| Seed medium (pH 7.2) | glucose 10g, ammonium sulfate 2.68g, yeast extract 20g, corn steep liquor 30 g/L, NaH₂PO₄2H₂O 4.5g, K₂HPO₄ 14.6g, MgSO47H2O 2g, Na2SO4 2 g/L (per 1L of distilled water) |

**[Table 7]**

| Strain | Fluorescence intensity | Relative fluorescence intensity |
|---|---|---|
| ATCC23857/pHT_P43_gfp | 279 | 100% |
| ATCC23857/pHT_Po2_dgfp | 296 | 106% |
| ATCC23857/pHT_Po2.1_gfp | 334 | 120% |
| ATCC23857/pHT_Po2.2_gfp | 352 | 126% |

As shown in Table 7, the Po2.1 and Po2.2 promoters exhibited promoter activity in *Bacillus subtilis,* and showed higher fluorescence intensity than P43 and Po2, which are known as strong conventional promoters.

### Example 2. Evaluation of target product production ability

### Example 2-1. Evaluation of L-threonine production ability

### Example 2-1-1. Construction of a pyc expression vector comprising Po2 or Po2 variant sequences

To evaluate the effects of Po2.1 and Po2.2, which showed high fluorescence intensity among the four Po2 mutant promoters, on threonine production, vectors were constructed as described below in which the *pyc* gene (Ncgl0659, SEQ ID NO: 21), an essential gene for the supply of precursors required for L-threonine production, is expressed under the control of the Po2.1, Po2.2, or Po2 promoter.

Specifically, a vector was first constructed to replace the native promoter of the chromosomal *pyc* gene with Po2 promoter variants. PCR was performed using the DNA of the pCES_Po2.1_gfp or pCES_Po2.2_gfp vector constructed in Example 1-1 as templates with primers of SEQ ID NOs: 22 and 23, respectively. After denaturation at 95 °C for 5 minutes, 30 cycles of denaturation at 95 °C for 30 seconds, annealing at 55 °C for 30 seconds, and extension at 72 °C for 30 seconds were performed, followed by a final extension at 72 °C for 7 minutes. As a result, DNA fragments containing the Po2.1 or Po2.2 promoter sequences, which can be inserted upstream of the *pyc* gene were obtained. For use as a control, PCR was performed in the same manner using pCES_Po2_gfp as a template to obtain a DNA fragment containing the Po2 promoter sequence.

In addition, PCR was performed using genomic DNA of *Corynebacterium glutamicum* ATCC13032 as a template with primers of SEQ ID NOs: 24 and 25, and SEQ ID NOs: 26 and 27. PCR was carried out under conditions of denaturation at 95 °C for 5 minutes, followed by 30 cycles of denaturation at 95 °C for 30 seconds, annealing at 55 °C for 30 seconds, and extension at 72 °C for 60 seconds, and a final extension at 72 °C for 7 minutes. As a result, a 524 bp DNA fragment corresponding to the upstream 5' region and a 525 bp DNA fragment corresponding to the downstream 3' region centered around the insertion site of the Po2.1, Po2.2, or Po2 promoter were obtained. As a result, a 524 bp DNA fragment of the 5' upstream region and a 525 bp DNA fragment of the 3' downstream region, centered on the o2.1, o2.2, and o2 promoter regions to be inserted, were obtained.

Using the three PCR products obtained above as templates, PCR was performed with primers of SEQ ID NOs: 24 and 27. After denaturation at 95 °C for 5 minutes, 30 cycles of denaturation at 95 °C for 30 seconds, annealing at 55 °C for 30 seconds, and extension at 72 °C for 90 seconds were performed, followed by a final extension at 72 °C for 7 minutes. As a result, a 1114 bp DNA fragment for replacing the native promoter region of the *pyc* gene with Po2.1, Po2.2, or Po2 promoters was amplified.

Thereafter, the pDC24 vector (SEQ ID NO: 43) and the 1110 bp DNA fragment were treated with the restriction enzyme Smal and cloned using an In-Fusion^{®} HD cloning kit (Clontech) to obtain plasmids. The vector in which the native promoter of the pyc gene was replaced with o2.1 was designated as "pDC24_o2.1_pyc," the vector with o2.2 as "pDC24_o2.2_pyc," and the control vector with o2 as "pDC24_o2_pyc." The primers of SEQ ID NOs: 22 to 27 used above are listed in Table 8 below.

**[Table 8]**

| SEQ IN NO. | Sequence (5'→3') |
|---|---|
| SEQ ID NO. 22 | AAGTTTAGAAATATTcaataatcgtgaattttggg |
| SEQ ID NO. 23 | GATGTGTGAGTCGACACtgttttgatctcctc |
| SEQ ID NO. 24 | AGCTCGGTACCCGAGCCCCATCCGTT |
| SEQ ID NO. 25 | tcacgattattgAATATTTCTAAACTT |
| SEQ ID NO. 26 | atcaaaacaGTGTCGACTCACACATCTT |
| SEQ ID NO. 27 | ACTCTAGAGGATCCCCACAAAGATGGGGTA |

### Example 2-1-2. Preparation of transformed strains

The recombinant vectors pDC24_Po2.1_pyc, pDC24_Po2.2_pyc, and pDC24_Po2_pyc constructed in Example 2-1-1 were each introduced into the threonine-producing strain *Corynebacterium glutamicum* KCCM12120P (U.S. Patent No. US 11,236,374 B2) by electroporation. Transformed strains were obtained on a selection medium containing kanamycin (25 mg/L), and were designated as "KCCM12120P::Po2.1_pyc", "KCCM1212OP::Po2.2_pyc", and "KCCM12120P::Po2_pyc", respectively.

### Example 2-1-3. Evaluation of threonine production ability of transformed strains

The transformed strains prepared in Example 2-1-2 were cultured by the following method, and threonine production was measured. KCCM12120P::Po2_pyc was used as a control strain.

Specifically, each transformed strain was inoculated into a 250 mL corner-baffled flask containing 25 mL of seed medium and shake-cultured at 30 °C and 200 rpm for 20 hours. Thereafter, 1 mL of the seed culture was inoculated into a 250 mL corner-baffled flask containing 24 mL of production medium and shake-cultured at 30 °C and 200 rpm for 48 hours. The compositions of the seed medium and the production medium are shown in Table 9 below.

**[Table 9]**

| Medium type | Composition |
|---|---|
| Seed medium (pH 7.0) | glucose 20 g, peptone 10 g, yeast extract 5 g, urea 1.5 g, KH₂PO₄ 4 g, K₂HPO₄ 8 g, MgSO₄ 7H2O 0.5 g, biotin 100 µg, thiamine HCl 1000 µg, calcium pantothenate 2000 µg, nicotinamide 2000 µg (per 1 L of distilled water) |
| Production Medium (pH 7.0) | glucose 30 g, (NH4)2SO4 12.5 g, soybean protein 2.5 g, Corn Steep Solids 5 g, urea 3 g, KH₂PO₄ 1 g, MgSO₄7H₂O 0.5 g, biotin 100 µg, thiamine hydrocholoride 1000 µg, calcium pantothenate 2000 µg, nicotinamide 3000 µg, CaCO₃ 30 g (per 1 L of distilled water) |

After completion of cultivation, L-threonine production ability was measured by HPLC.

The percentage increase (%) in L-threonine concentration in the culture broth of each strain is shown in Table 10 below.

**[Table 10]**

| Strain | Increase in L-threonine concentration (%) | L-threonine concentration (g/L) |
|---|---|---|
| Control (KCCM12120P::Po2_pyc) | 100% | 1.5 |
| KCCM12120P::Po2.1_pyc | 108.84% | 1.63 |
| KCCM12120P::Po2.2_pyc | 111.56% | 1.67 |

As shown in Table 10, it was confirmed that the introduction of the variant promoters of Po2 led to an increase in pyc activity, thereby increasing threonine production. These results demonstrated that Po2.1 and Po2.2 are effective in enhancing threonine production.

### Example 2-2. Evaluation of O-acetyl homoserine production ability

### Example 2-2-1. Construction of a metX expression vector comprising Po2 or Po2 variant sequences

To evaluate the effect of Po2 promoter variants on O-acetyl homoserine production ability, vectors were constructed as described below in which *metX* gene (NCgl0624, SEQ ID NO: 28), an important gene for O-acetyl homoserine production, is expressed under the control of the Po2.1, Po2.2, or Po2 promoter.

Specifically, PCR was performed using the DNA of the pCES_Po2.1_gfp or pCES_Po2.2_gfp vector constructed in Example 1-1 as templates with primers of SEQ ID NOs: 2 and 29, respectively. PCR was carried out by denaturation at 94 °C for 5 minutes, followed by 30 cycles of denaturation at 94 °C for 30 seconds, annealing at 55 °C for 30 seconds, and extension at 72 °C for 1 minute, and a final extension at 72 °C for 5 minutes, thereby obtaining DNA fragments containing the o2.1 or o2.2 promoter sequence. For use as a control, PCR was performed in the same manner using pCES_Po2_gfp as a template to obtain a DNA fragment comprising the Po2 promoter sequence.

In addition, PCR was performed using genomic DNA of *Corynebacterium glutamicum* ATCC13032 as a template with primers of SEQ ID NOs: 30 and 31 to obtain a DNA fragment comprising *metX.* PCR was carried out by denaturation at 94 °C for 5 minutes, followed by 30 cycles of denaturation at 94 °C for 30 seconds, annealing at 55 °C for 30 seconds, and extension at 72 °C for 2 minutes, and a final extension at 72 °C for 5 minutes.

Further, in order to introduce Po2.1, Po2.2, or Po2 and *metX* into the *Escherichia coli-Corynebacterium* shuttle vector pCES208, PCR was performed using the pCES208 vector as a template with primers of SEQ ID NOs: 10 and 11 to prepare the vector. PCR was carried out by denaturation at 94 °C for 5 minutes, followed by 30 cycles of denaturation at 94 °C for 30 seconds, annealing at 55 °C for 30 seconds, and extension at 72 °C for 5 minutes, and a final extension at 72 °C for 5 minutes.

Thereafter, the Po2.1, Po2.2, or Po2 DNA fragment obtained above and the ORF of the *metX* gene were operably linked into the pCES208 vector using an In-Fusion^{®} HD cloning kit (Clontech) to construct recombinant vectors in which Po2.1, Po2.2, or Po2 was respectively linked to *metX.* The resulting vectors were designated as "pCES_Po2.1_MetX", "pCES_Po2.2_MetX", and "pCES_Po2_MetX".

The primers of SEQ ID NOs: 29 to 31 used above are shown in Table 11 below.

**[Table 11]**

| SEQ IN NO. | Sequence (5'→3') |
|---|---|
| SEQ ID NO. 29 | CTGAAGGCGCGAGGGTGGGCATtgttttgatctcctccaataat |
| SEQ ID NO. 30 | attattggaggagatcaaaacaATGCCCACCCTCGCGCCTTCAG |
| SEQ ID NO. 31 | TGATATCGAATTCCTGCAGCCCTTAGATGTAGAACTCGATGT AG |

### Example 2-2-2. Preparation of transformed strains

The recombinant vectors "pCES_Po2.1_MetX", "pCES_Po2.2_MetX", and "pCES_Po2_MetX" constructed in Example 2-2-1 were each introduced into the acetyl homoserine-producing strain *Corynebacterium glutamicum* KCCM12634P (EP 4050022 A1) by electroporation. Transformed strains were obtained on a selection medium containing kanamycin (25 mg/L), and were designated as "KCCM12634P::Po2.1_metX", "KCCM12634P::Po2.2_metX", and "KCCM12634P::Po2_metX", respectively.

### Example 2-2-3. Evaluation of O-acetyl homoserine production ability of transformed strains

The transformed strains prepared in Example 2-2-2 were cultured by the following method, and O-acetyl homoserine production was measured. KCCM12634P::Po2_metX was used as a control strain.

Specifically, each strain was inoculated with one inoculation loop into a 250 mL corner-baffled flask containing 25 mL of production medium and shake-cultured at 37 °C and 200 rpm for 20 hours. The composition of the production medium is shown in Table 12 below.

**[Table 12]**

| Medium type | Composition |
|---|---|
| Production medium (pH 7.2) | glucose 30 g, KH₂PO₄ 2 g, urea 3 g, |
| | (NH₄)₂SO₄ 40 g, peptone 2.5 g, CSL(Sigma) 5 g(10 ml), MgSO₄.7H₂O 0.5 g, methionine 400 mg, Leucine 400 mg, CaCO₃ 20 g (per 1 L of distilled water) |

After completion of cultivation, the concentration of O-acetyl homoserine was analyzed by HPLC. The percentage increase in O-acetyl homoserine concentration measured in each strain is shown in Table 13 below.

**[Table 13]**

| Strain | Increase in O-acetyl homoserine concentration (%) | O-acetyl homoserine concentration (g/L) |
|---|---|---|
| Control (KCCM12634P::Po2_metX) | 100% | 2.2 |
| KCCM12634P::Po2.1_metX | 113.66% | 2.5 |
| KCCM12634P::Po2.2_metX | 116.77% | 2.6 |

As shown in Table 13, introduction of the Po2 variant promoters increased *metX* activity, resulting in increased O-acetyl homoserine production. From this, it was confirmed that Po2.1 and Po2.2 are effective in increasing O-acetyl homoserine production.

### Example 2-3. Evaluation of L-valine production ability

### Example 2-3-1. Construction of an ilvE expression vector comprising Po2 or Po2 variant sequences

To evaluate the effect of Po2 promoter variants on valine production ability, vectors were constructed as described below in which *ilvE* (Ncgl2123, SEQ ID NO: 32), encoding branched-chain amino acid aminotransferase, a key enzyme in valine biosynthesis, is expressed under the control of the Po2.1, Po2.2, or Po2 promoter.

Specifically, a vector was first constructed to replace the native promoter of the chromosomal *ilvE* gene with the Po2.1 or Po2.2 promoter. PCR was performed using the DNA of the pCES_Po2.1_gfp or pCES_Po2.2_gfp vector prepared in Example 1-1 as templates with primers of SEQ ID NOs: 33 and 34, respectively. After denaturation at 95 °C for 5 minutes, 30 cycles of denaturation at 95 °C for 30 seconds, annealing at 55 °C for 30 seconds, and extension at 72 °C for 30 seconds were performed, followed by a final extension at 72 °C for 7 minutes. As a result, DNA fragments containing the Po2.1 or Po2.2 promoter sequence which can be inserted upstream of the *ilvE* gene were obtained. For use as a control, PCR was performed in the same manner using pCES_Po2_gfp as a template to obtain a DNA fragment comprising the Po2 promoter sequence.

In addition, PCR was performed using chromosomal DNA of *Corynebacterium glutamicum* ATCC14067 as a template with primers of SEQ ID NOs: 35 and 36, and SEQ ID NOs: 37 and 38. PCR was carried out by denaturation at 95 °C for 5 minutes, followed by 30 cycles of denaturation at 95 °C for 30 seconds, annealing at 55 °C for 30 seconds, and extension at 72 °C for 60 seconds, and a final extension at 72 °C for 7 minutes. As a result, a 529 bp DNA fragment of the 5' upstream region and a 525 bp DNA fragment of the 3' downstream region, centered on the o2.1, o2.2, and o2 promoter regions to be inserted, were obtained.

Using the three PCR products obtained above as templates, PCR was performed with primers of SEQ ID NOs: 35 and 38. After denaturation at 95 °C for 5 minutes, 30 cycles of denaturation at 95 °C for 30 seconds, annealing at 55 °C for 30 seconds, and extension at 72 °C for 90 seconds were performed, followed by a final extension at 72 °C for 7 minutes. As a result, a 1114 bp DNA fragment for replacing the native promoter region of the *ilvE* gene with the o2.1, o2.2, or o2 promoter was amplified.

Thereafter, the pDC24 vector (SEQ ID NO: 43) and the 1114 bp DNA fragment were treated with the restriction enzyme Smal and cloned using an In-Fusion^{®} HD cloning kit (Clontech) to obtain plasmids. The vector in which Po2.1 was introduced in place of the native promoter of the *ilvE* gene was designated as "pDC24_Po2.1_ilvE", the vector in which Po2.2 was introduced was designated as "pDC24_Po2.2_ilvE", and the control vector in which Po2 was introduced was designated as "pDC24_Po2_ilvE". The vector in which the native promoter of the *ilvE* gene was replaced with Po2.1 was designated as "pDC24_Po2.1_ilvE," the vector with Po2.2 as "pDC24_Po2.2_ilvE," and the control vector with Po2 as "pDC24_Po2_ilvE."

The primers of SEQ ID NOs: 33 to 38 used above are shown in Table 14 below.

**[Table 14]**

| SEQ IN NO. | Sequence (5'→3') |
|---|---|
| SEQ ID NO. 33 | TCATCCCATAAAATcaataatcgtgaattttgg |
| SEQ ID NO. 34 | TAATGACGTCATtgttttgatctcctcc |
| SEQ ID NO. 35 | TTCGAGCTCGGTACCCCCGTGCCGGAAGCG |
| SEQ ID NO. 36 | ttcacgattattgATTTTATGGGATGAATAGC |
| SEQ ID NO. 37 | ggagatcaaaacaATGACGTCATTAGAGTTC |
| SEQ ID NO. 38 | TAGAGGATCCCCATGACCAGGAACTTG |

### Example 2-3-2. Preparation of transformed strains

The recombinant vectors pDC24_Po2.1_ilvE, pDC24_Po2.2_ilvE, and pDC24_Po2_ilvE constructed in Example 2-3-1 were each introduced into the valine-producing strain *Corynebacterium glutamicum* KCCM11201 P (U.S. Patent No. US 8,465,962 B2) by electroporation. Transformed strains were obtained on a selection medium containing kanamycin (25 mg/L), and were designated as "KCCM11201P::Po2.1_ilvE", "KCCM11201P::Po2.2_ilvE", and "KCCM11201P::Po2_ilvE", respectively.

### Example 2-3-3. Evaluation of valine production ability of transformed strains

The transformed strains prepared in Example 2-3-2 were cultured by the following method, and valine production was measured. KCCM12120P::P₀2_ilvE was used as a control strain.

Specifically, each strain was inoculated into a 250 mL corner-baffled flask containing 25 mL of production medium and shake-cultured at 30 °C and 200 rpm for 72 hours. The composition of the production medium used is shown in Table 15 below.

**[Table 15]**

| Medium type | Composition |
|---|---|
| Production medium (pH 7.2) | glucose 100 g, ammonium sulfate 40 g, soybean protein 2.5 g, Corn Steep Solids 5 g, urea 3 g, dipotassium phosphate 1 g, magnesium sulfate heptahydrate 0.5 g, biotin 100 µg, thiamine-HCl 1 mg, calcium pantothenate 2 mg, nicotinamide 3 mg, calcium carbonate 30 g (per 1 L of distilled water) |

After completion of cultivation, L-valine production ability was measured by HPLC.

The percentage increase (%) in L-valine concentration in the culture broth of each strain is shown in Table 16 below.

**[Table 16]**

| Strain | Increase in L-valine concentration (%) | L-valine concentration (g/L) |
|---|---|---|
| KCCM11201P::Po2_ilvE | 100% | 2.7 |
| KCCM11201P::Po2.1_ilvE | 109.62% | 3.0 |
| KCCM11201P::Po2.2_ilvE | 114.38% | 3.1 |

As shown in Table 16, introduction of the Po2 variant promoters increased *ilvE* activity, resulting in increased valine production. From this, it was confirmed that Po2.1 and Po2.2 are effective in increasing valine production.

From the above description, those skilled in the art to which the present invention pertains will understand that the present invention can be implemented in other specific forms without changing the technical spirit or essential features thereof. In this regard, it should be understood that the examples described above are exemplary and not restrictive in all aspects. The scope of the present invention should be interpreted as including all changed or modified forms derived from the meaning and scope of the claims described below and equivalent concepts thereof rather than the detailed description above.

## Claims

1. A polynucleotide comprising any one nucleic acid sequence selected from the group consisting of SEQ ID NO: 39 and SEQ ID NO: 40.

2. The polynucleotide of claim 1, wherein the polynucleotide has promoter activity.

3. An expression cassette comprising the polynucleotide of claim 1 and a target gene.

4. A microorganism comprising
the polynucleotide of claim 1 or the expression cassette of claim 3.

5. The microorganism of claim 4, wherein the microorganism is a microorganism of the genus *Corynebacterium,* a microorganism of the genus *Escherichia,* or a microorganism of the genus *Bacillus.*

6. A method for producing a target product, comprising culturing the microorganism of claim 4 in a medium.

7. The method for producing a target product of claim 6, further comprising recovering a target product from the medium or the microorganism resulting from the culturing.

8. The method for producing a target product of claim 6, wherein the target product is at least one selected from the group consisting of amino acids, amino acid derivatives, nucleic acids, nucleic acid derivatives, vitamins, vitamin derivatives, proteins, fatty acids, fatty acid derivatives, organic acids and other metabolites.

9. The method for producing a target product of claim 6, wherein the target product is one or more selected from the group consisting of threonine, O-acetyl homoserine, and valine.

10. The method for producing a target product of claim 6, wherein the microorganism is a microorganism of the genus *Corynebacterium,* a microorganism of the genus *Escherichia,* or a microorganism of the genus *Bacillus.*
